# EUROPEAN PATENT APPLICATION

(11) **EP 3 539 576 A1**
(43) Date of publication of application: **18.09.2019**
(21) Application number: 18161631.9
(22) Date of filing: 13.03.2018
(51) Int. Cl.: A61L 27/20, A61L 27/26, A61L 27/48, A61L 27/52, A61L 27/58

(54) **HYDROGEL COMPOSITES COMPRISING CHITOSAN AND CELLULOSE NANOFIBERS**

(71) Applicant: Albert-Ludwigs-Universität Freiburg, 79085 Freiburg (DE); Université Claude Bernard Lyon 1, 69100 Villeurbanne (FR); Centre National de la Recherche Scientifique - CNRS, 75016 Paris (FR); Institut Enseignement Supérieur et Recherche en Alimentation Santé Animale Sciences Agronomiques et Environnement (Vet Agro Sup), 69280 Marcy-l'Etoile (FR)
(72) Inventor: OSORIO MADRAZO, Anayancy, 79110 Freiburg (DE); DAVID, Laurent, 69004 Lyon (FR); MONTEMBAULT, Alexandra, 42800 Rive-de-Gier (FR); VIGUIER, Eric, 69160 Tassin-la-Demi-Lune (FR); CACHON, Thibaut, 69280 Marcy-l'Étoile (FR)
(74) Representative: Kalhammer, Georg

(57) **Abstract**

The present invention relates to an aqueous or hydro-alcoholic suspension of chitosan, dispersed cellulose nanofibers and an acid, which is suitbale for obtaining a hydrogel composite comprising a network of chitosan and cellulose nanofibers. The suspension of the invention is useful for tissue regeneration and the hydrogel of the invention is useful as bioresorbable and biocompatible implant.

## Description

The present invention relates to an aqueous or hydro-alcoholic suspension of cellulose nanofibers and chitosan and a hydrogel composite obtainable therefrom, which can be used for tissue regeneration and for the provision of bioresorbable and biocompatible implants.

US 2015/0065454 A1 discloses a homogeneous aqueous solution of chitosan, which is capable of forming crystalline particles of chitosan after injection. The compositions containing the homogeneous aqueous solution disclosed in this document can be used as medical device, in particular as bioresorbable implant.

Chitosan solutions as e.g. disclosed in US 2015/0065454 A1 easily form a hydrogel. Such hydrogels are however known to exhibit poor mechanical properties, limiting their use as structural materials. It is challenging with such a material to achieve long-term support for the repairing and regeneration of native cartilaginous/meniscus tissues and to use it as sutured implant, which is important in surgery treatments for defect meniscus and cartilaginous tissues and other connective tissues (such as tendon, dermis, annulus fibrosus tissue comprising the intervertebral disk).

Alternatively, hyaluronic acid has been used for injection into defect tissues. Hyaluronic acid is advantageous in terms of an immediate mechanical filling effect and an absence of inflammatory phenomena. However, this biocompatibility of hyaluronic acid is also associated with rapid biodegradation, making the product unsatisfactory for long-term use.

More invasive techniques rely on the reconstruction of a joint surface by mosaicplasty and implantation of hydrogels to replace defective cartilage (agarose/alginate systems). The gain and feedback of such mosaicplasty is also questionable and many initial developments have been abandoned because of the poor quality of reconstructed tissues.

The present invention attempts to overcome these disadvantages of the prior art by providing a suspension as defined in claim 1, i.e. a suspension suitable for forming a hydrogel composite, the suspension comprising water (aqueous suspension) or a mixture of water and an alcohol (hydro-alcoholic suspension) as the solvent, cellulose nanofibers dispersed in said solvent, chitosan and an acid.

The present invention overcomes the disadvantages of the prior art by furthermore providing a hydrogel as defined in claim 9, i.e. a hydrogel obtained by gelation of the suspension of the invention.

Preferred embodiments and uses of the suspension and the hydrogel of the present invention are specified in the subclaims and will be explained in the following description of the invention.

The term "suspension", as used herein, refers to a heterogeneous mixture, which in general means a mixture of at least two components in different state of matter. In the context of the present invention, the term "suspension" in particular refers to a mixture of a solvent, said solvent being water or a water/alcohol mixture, and a solid material dispersed in the solvent. Said solid material in the context of the present invention are cellulose nanofibers, which are dispersed in water or a water/alcohol mixture. In the context of the present invention, the solvent further comprises an acid dissolved therein, which makes the solvent acetic, allowing dissolving chitosan therein. The suspension of the present invention thus comprises dispersed cellulose nanofibers and dissolved chitosan.

The "cellulose nanofibers", as used in the context of the present invention, refers to nanosized cellulose. Typical widths of the fibers are from 3 to 20 nanometers with lengths within 50 nm to the micrometer range, resulting in high aspect ratios (length to width ratio higher than 20) of the fibers. The cellulose nanofibers used in the present invention are not oxidized cellulose nanofibers, in particular not TEMPO-oxidized cellulose nanofibers.

The term "wt.-%", as used herein, is an abbreviation for "weight percent", and refers to the weight amount of a component in relation to the weight amount of a total of components. It is indicated herein, which component in relation to which total of components is meant. The abbreviation wt.-% is an alternative for the abbreviation "(w/w)".

The term "hydrogel" in general refers to a network of hydrophilic polymer chains which are dispersed in water resulting in a solid material with high water content (usually higher than 70%). The rheological definition of a hydrogel is a highly hydrated material easy to handle without flowing and exhibiting viscoelastic properties such as a storage elastic modulus G' or E' that is much larger than the corresponding loss modulus (G" or E"), typically G'>10G" or E'>10E". In the context of the present invention, the terms "hydrogel" or, alternatively, "hydrogel composite" refers to a network of cellulose fibers and chitosan further comprising water, which is obtained from the suspension of the invention by gelation.

The suspension of the present invention can be prepared via an aqueous route. A typical procedure of the aqueous route includes mixing of an aqueous slurry of cellulose fibers with an aqueous dispersion of chitosan powder. In order to bring the chitosan into solution, an organic is added. The resulting (aqueous) suspension comprises water as the solvent, cellulose nanofibers dispersed in said solvent, chitosan and an acid.

The suspension of the present invention can also be prepared via a hydro-alcoholic route. A typical procedure of the hydro-alcoholic route includes mixing of an aqueous slurry of cellulose fibers with an aqueous dispersion of chitosan powder. In order to bring the chitosan into solution, an organic is added, followed by the addition of an alcohol after the chitosan has completely dissolved. The resulting (hydro-alcoholic) suspension thus comprises water and an alcohol as the solvent, cellulose nanofibers dispersed in said solvent, chitosan and an acid.

"Hydro-alcoholic" means that the solvent is a mixture of water and an alcohol, wherein the alcohol is selected from the group consisting of ethanol, propanol, propanediols such as 1,2-propanediol and 1,3-propanediol, 1-butanol, and 2-butanol, butanediols and glycerol. A preferred alcohol for the provision of a hydro-alcoholic suspension is 1,2-propanediol. The alcohol in the hydro-alcoholic route is typically added in a weight amount equal to the weight of the aqueous suspension.

The aqueous route and the hydro-alcoholic route for the preparation of the suspension of the present invention are also illustrated in Example 1 described herein below.

As mentioned above, the suspension of the invention contains an acid, which ensures a protonation of the amine groups of chitosan and thereby an increase of the hydrophilicity of chitosan at a pH below 6.5, preferably below 6, more preferably from 3 to 5, particularly preferably 4.5 (±0.2). The concentration of the acid in the suspension of the invention depends on the amine moieties in the chitosan chains. In the present invention, stoichiometric amounts of the acid in relation to the amine moieties in the chitosan chains are used. This ensures protonation and solubilization of chitosan, and avoids the use of an excess of acid. The acid used in the suspension of the present invention is preferably an organic acid, and particularly preferably acetic acid.

By raising the pH of the suspension of the present invention, the suspension is converted into a hydrogel composite comprising cellulose nanofibers and chitosan. This can e.g. be achieved by adding NaOH or NH₃ to the suspension of the invention, which raises the pH above the apparent pKa of amine groups of chitosan (from 6.2 to 6.5 depending of the DA ranging from 0 to 65%) resulting in the gelation of the suspension, in particular of the chitosan contained therein.

The combination of cellulose nanofibers and chitosan in the hydrogel composite of the present invention results in an enhancement of the mechanical properties of the hydrogel composite. Moreover, the combination of cellulose nanofibers and chitosan increases the suturability of the hydrogel composite (wherein the suturability is as defined in WO 2015/092289 A1), which is important in surgery treatments for meniscus and cartilaginous tissues. In the hydrogel composite of the present invention, the solubilized chitosan and the dispersed cellulose nanofibrils interact, the cellulose nanofibrils interact (directly or mediated by chitosan) and chitosan chain interact for the hydrogel, which ensures the formation of a strong physical network, with an elastic modulus in the range of 5 MPa - 1 kPa, which is beneficial for the mechanical properties and the suturability of the hydrogel composite of the present invention. This advantageous in view of a long-term use of the hydrogels in tissue engineering or in resorbable and biocompatible implants or degradable materials with high mechanical properties.

The concentration of chitosan in the suspension of the present invention can be up to 15 wt.-%, but is preferably from 1 to 5 wt.-%, and more preferably from 2 to 4 wt.-%, each based on the total weight of the suspension. In general, higher chitosan concentrations are technically feasible for chitosan with a lower molecular weight. The chitosan used in the present invention has a molecular weight from 50 to 1000 kg/mol, preferably from 400 to 800 kg/mol, and particularly preferably about 650 kg/mol. The Degree of Acetylation (DA) of chitosan ranges from 0 to 70%, preferably from 0 to 15%.

The content of the cellulose nanofibers in the suspension of the present invention can be up to 5 wt.-%, but is preferably from 0.01 to 2 wt.-%, more preferably from 0.02 to 0.6 wt.-%, each based on the total weight of the suspension.

In preferred embodiments of the suspension of the invention, the cellulose nanofiber content is from 0.02 to 2 wt.-% and the chitosan concentration is from 1 to 4 wt.-%, each based on the total weight of the suspension. In more preferred embodiments of the suspension according to the invention, the cellulose nanofiber content from 0.02 to 0.6 wt.-%, and the concentration of chitosan is from 2 to 3 wt.-%, each based on the total weight of the suspension.

The suspension of the present invention can be turned into a hydrogel composite comprising chitosan and dispersed cellulose nanofibers by means of neutralizing the suspension of the invention, i.e. raising the pH of the suspension, which can be achieved by means of addition NaOH or NH₃ to the suspension. If the suspension is injected into damaged tissue of a patient, a first increase of pH of the suspension is achieved by means of addition of NaOH or by dialysis against a buffer solution with osmolarity of 300 mOs. Then, gelation of the suspension takes place by means of contact with the body fluids.

For achieving a particularly good mechanical performance of a hydrogel derived from an hydro-alcoholic suspension of the invention, it is preferred that the concentration of chitosan in the hydro-alcoholic suspension is from 2 to 3 wt.-%, based on the total weight of the suspension. The enhancement effect on the mechanical stability introduced by the cellulose nanofibers is significant in that case up to a concentration of cellulose nanofibers of 0.45 wt.-%, based on the total weight of the suspension.

For achieving a particularly good mechanical performance of a hydrogel derived from an aqueous suspension of the invention, the chitosan concentration is preferably from 2 to 4 wt.-%, based on the total weight of the suspension. In that case, the reinforcement effect introduced by the cellulose nanofibers is significant up to a concentration of the cellulose nanofibers of 0.6 wt.-%, based on the total weight of the suspension.

The hydrogels according to the present invention have an equilibrium storage modulus G' of at least 2600 Pa, and preferably between 2600 to 5000 Pa, more preferably 2800 to 4500 Pa, even more preferably between 3000 and 4000 Pa. The storage modulus G' is to be measured as described in Example 3 herein below.

For achieving particularly good hydrogel equilibrium storage moduli G' for hydrogels obtained from suspensions prepared via the hydro-alcoholic route, the concentration of chitosan in the hydro-alcoholic suspension is from 2 to 3 wt.-% and the content of cellulose nanofibers is from 0.2 to 0.6 wt.%, based on the total weight of the suspension. For hydrogels obtained from such suspensions, the hydrogel equilibrium storage moduli G' are generally greater than 2600 Pa.

For achieving particularly good hydrogel storage moduli G' for hydrogels obtained from suspensions prepared via the aqueous route, the concentration of chitosan in the hydro-alcoholic suspension is from 2 to 3 wt.-% and the content of cellulose nanofibers is from 0.02 to 0.6 wt.%, based on the total weight of the suspension. For hydrogels obtained from such suspensions, the hydrogel equilibrium storage moduli G' are generally greater than 3300 Pa.

The hydrogels according to the present invention have a normalized ultimate suture force (ultimate suture force normalized by the hydrogel patch thickness) of at least 0.03 N.mm⁻¹, and is preferably between 0.05 and 0.3 N.mm⁻¹, more preferably between 0.05 and 0.2 N.mm⁻¹. The normalized ultimate suture force is determined as described in Example 4.

Such normalized ultimate suture forces are preferably achieved with hydrogels obtained from suspensions comprising 2 to 4 wt.-% chitosan, more preferably 2 % chitosan, and 0.2 to 0.6 wt.-% cellulose nanofibers.

The normalized ultimate suture force tends to be higher for hydrogels obtained from suspensions prepared via the aqueous route. In that case, the normalized ultimate suture force is at least 0.07 N.mm⁻¹, and is preferably between 0.07 and 0.3 N.mm⁻¹, and more preferably between 0.07 and 0.2 N.mm⁻¹.

The suspension of the present invention can be used for tissue regeneration, in particular for regeneration of cartilaginous, meniscus or other connective tissue selected from tendon, dermis, and annulus fibrosus tissue comprising the intervertebral disk.

For achieving tissue regeneration, the suspension of the invention is injected for example into an osteochondral defect or a meniscus/cartilage lesion to promote tissue regeneration. The injected suspension then fills the defect and seal the defect with good bonding to the adjacent tissue and forms a hydrogel in the body. The advantage of this method is that it is mini-invasive and allows restoration of mobility over the time needed for the regeneration of the tissue, which can take up to 6 months, depending on the targeted tissue to be regenerated.

The suspension of the present invention can also be extruded and the gelation can be integrated in a fiber spinning process to obtain chitosan-based fibers as hydrogel fibers or solid fibers with enhanced mechanical properties for knitted textile implants or suture threads, which are highly bioresorbable and biocompatible.

The invention shall be illustrated by means of the following Examples. These are not intended to limit the scope of the present invention.

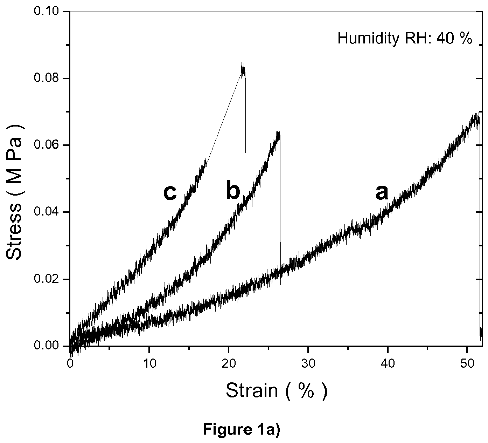

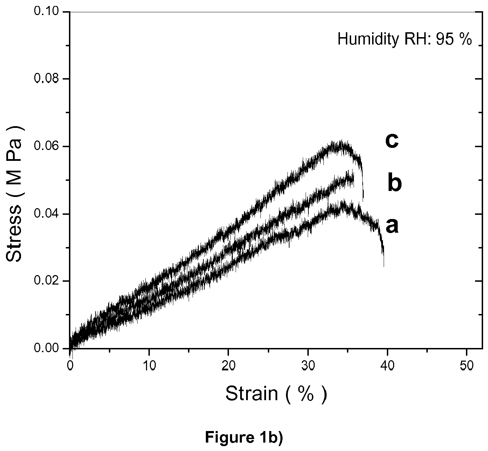

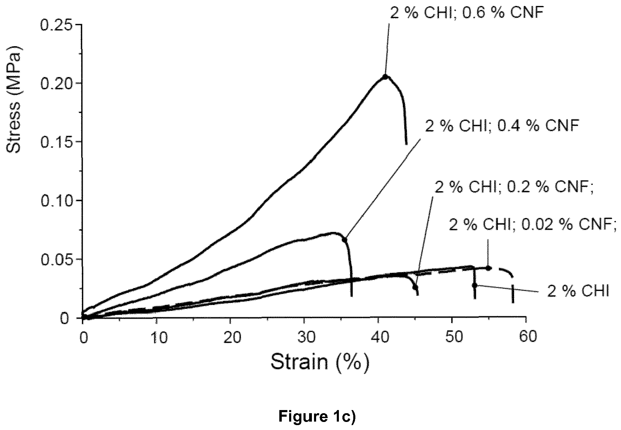

**Figure 1a****),** **1b****),** and **1c****)** show the results of microtensile tests of hydrogel composites with different contents of chitosan and cellulose nanofibers at different relative humidities. **Figure 1a****):** CHI/CNF: 3/0 (curve a), 3/0.30 (curve b), 3/0.45 (curve c) at 40% relative humidity (RH); **Figure 1b****):** CHI/CNF: 3/0 (curve a), 3/0.30 (curve b), 3/0.45 (curve c) at 95% relative humidity (RH); **Figure 1c****):** CHI/CNF: 2/0, 2/0.02, 2/0.20, 2/0.40, 2/0.60 at 40% relative humidity.

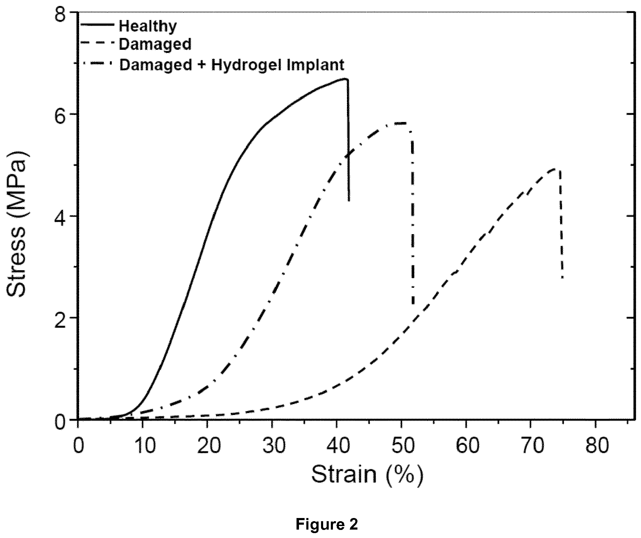

**Figure 2** shows compression stress-strain curves observed as mechanical response of a healthy, a fenestred damaged disc and a fenestrated hydrogel-implanted intervertebral disc. Physiological loading ranges between 1 and 4 MPa.

### Example 1: Exemplary procedure for the preparation of chitosan/cellulose nanofiber suspensions and hydrogels

a) Suspensions and hydrogels obtained from the aqueous route:
   Cellulose nanofibers commercially available according to reference [Fumagali, M, Ouhab, D., Molina Boisseau S., Heux L, Versatile gaz phase reactions for for surface to bulk esterification of cellulose microfibers aerogels, Biomacromoleculmes 14, 21013, pp3246-3255] in the form an aqueous slurry and at a concentration of 0.4 wt.-% were mixed with chitosan powder (Mw = ca. 600 kg/mol, DA=3%) dispersed in water at a concentration of 2 wt.-%. This mixture was sonicated (Ultrasonic homogenizer SonoPlus HD200 -Bandelin, Germany, at 45% amplitude for 5 minutes). The mixture was then mechanically stirred during 1 hour. Then acetic acid was added at a stoichiometric concentration in relation to the amine groups of chitosan (n(acetic Acid)/n(-NH₂)=1 mol/mol, where n(acetic acid) is the number of moles of acetic acid, and n(-NH₂) is the number of moles of glucosamine units) resulting in a pH close to 4.5. The mixture was mechanically stirred in a close reactor during 5 hours to achieve complete solubilization of chitosan.
   Hydrogels were obtained by neutralization of the suspension, poured in Petri dishes, using NaOH 2M during 1 hour. The hydrogels are then washed in deionized water until neutrality of the washing bath and removal of the neutralization salts.
b) Suspensions and hydrogels obtained from the hydro-alcoholic route:
   Cellulose nanofibers commercially available according to reference [Fumagali, M, Ouhab, D., Molina Boisseau S., Heux L, Versatile gaz phase reactions for surface to bulk esterification of cellulose microfibers aerogels, Biomacromoleculmes 14, 21013, pp3246-3255] in the form an aqueous slurry and at a concentration of 0.8 wt.-% were mixed with chitosan powder (Mw = ca. 600 kg/mol, DA=3%) dispersed in water at a concentration of 4 wt.-%. This mixture was sonicated (Ultrasonic homogenizer SonoPlus HD200 -Bandelin, Germany, at 45% amplitude for 5 minutes). The mixture was then mechanically stirred during 1 hour. Then acetic acid was added at a stoichiometric concentration in relation to the amine groups of chitosan (n(acetic acid)/n(-NH₂)=1 mol/mol, where n(acetic acid) is the number of moles of acetic acid, and n(-NH₂) is the number of moles of glucosamine units) resulting in a pH close to 4.5. The mixture was mechanically stirred in a close reactor during 5 hours to achieve complete solubilization of chitosan. Afterwards, a weight of 1,2-propanediol equal to the weight of the aqueous suspension was added. Thus, the final chitosan concentration was 3 wt.-% and the cellulose nanofibers content was 0.45 wt.-%.

Hydrogels were obtained by neutralization of the hydro-alcoholic suspension, poured in Petri dishes, using NaOH 2M during 1 hour. The hydrogels are then washed in deionized water until neutrality of the washing bath and removal of the neutralization salts, base and alcohol.

### Example 2: Microtensile tests

In this example, hydrogel composites obtained by gelation of aqueous our hydro-alcoholic suspensions comprising different contents of dispersed cellulose fibers (CNFs) and chitosan (CHI) were subjected to tensile mechanical tests performed with a home-made microtensile test machine equipped with a load cell of 50N. The cross-head speed was 1 micrometer/second (**Figure 1a****), b)**) or 8 micrometer/second (**Figure 1c**). The experiments were performed in a chamber that allowed humidity control (from 23 to 95% relative humidity (RH)). The results are shown in **Figures 1a****),** **1b****) and** **1c****).**

Hydrogel composites as shown in the following Tables 1-3 were examined:

**Table 1: Hydrogels composites according to Figure 1a):**

| **Entry** | **Relative humidity** | **CHI content [wt.-%]** | **CNF content [wt.-%]** | **Preparation route of the suspension** |
|---|---|---|---|---|
| **1** | 40% | 3 | 0.00 | hydro-alcoholic |
| **2** | 40% | 3 | 0.30 | hydro-alcoholic |
| **3** | 40% | 3 | 0.45 | hydro-alcoholic |

**Table 2: Hydrogels composites according to Figure 1b):**

| **Entry** | **Relative humidity** | **CHI content [wt.-%]** | **CNF content [wt.-%]** | **Preparation route of the suspension** |
|---|---|---|---|---|
| **1** | 95% | 3 | 0.00 | hydro-alcoholic |
| **2** | 95% | 3 | 0.30 | hydro-alcoholic |
| **3** | 95% | 3 | 0.45 | hydro-alcoholic |

The "hydro-alcoholic" preparation route referred to in **Table 1** and **2** refers to a water/1,2-propanediol mixture .

**Table 3: Hydrogels composites according to Figure 1c):**

| **Entry** | **Relative humidity** | **CHI content [wt.-%]** | **CNF content [wt.-%]** | **Preparation route of the suspension** |
|---|---|---|---|---|
| **1** | 40% | 2 | 0.00 | aqueous |
| **2** | 40% | 2 | 0.02 | aqueous |
| **3** | 40% | 2 | 0.20 | aqueous |
| **4** | 40% | 2 | 0.40 | aqueous |
| **5** | 40% | 2 | 0.60 | aqueous |

For hydrogel composites obtained from gelation of hydro-alcoholic suspensions of dispersed cellulose nanofibers and chitosan, the enhancement effect of the cellulose nanofibers was significant for CNF contents of 0.45 wt.-% at both relative humidities of 40% and 95% (**Figures 1a****)** and **1b****)**). Further increasing the content of the cellulose nanofibers led to a slight decrease of stiffness.

For hydrogels obtained from an aqueous suspension of dispersed cellulose nanofibers and chitosan, a significant reinforcement effect was also observed at higher CNF contents of up to 0.6 wt.-% (**Figure 1c****)**).

The results show that the CNFs provide reinforcement to the chitosan hydrogel matrix by using both processing methods, i.e. the hydro-alcoholic route or the aqueous route. The mechanical performance is related to the chitosan (CHI) concentration, the CNF content, the processing route and the environmental humidity.

### Example 3: Rheological measurements

Dynamic-mechanical rheological measurements were carried out at room temperature by using an ARES rheometer (TA Instruments) operating with a plate-plate geometry (diameter 25 mm). The strain amplitude was monitored to ensure the measurements were carried out within the linear viscoelastic region (maximum applied strain = 5 × 10⁻³), resulting in storage modulus (G') and loss modulus (G") independent of the strain amplitude. The gap distance between the two plates was around 1.0 mm. Angular frequency sweep measurements were then carried out in the range from 100 rad s⁻¹ down to 0.05 rad s⁻¹. Such analyses were repeated three times for each hydrogel. The characterization of CHI/CNF hydrogel composites obtained from gelation of aqueous or hydro-alcoholic suspensions comprising different contents of dispersed cellulose fibers (CNFs) and chitosan (CHI) were carried out in order to study the evolution of storage and loss moduli.

The following **Table 4** reports the hydrogel equilibrium storage modulus G', which corresponds to the plateau value of G' measured at low angular frequency, and on loss modulus G" determined by rheological measurements of cellulose nanofiber (CNF)-filled chitosan hydrogel composites of different compositions.

**Table 4**

| **Entry** | **Preparation route of the suspension** | **CHI Content [wt.-%]** | **CNF Content [wt.-%]** | **G'[Pa]** | **G" [Pa]** | **G"/G'** |
|---|---|---|---|---|---|---|
| **1** | hydro-alcoholic | 2 | 0 | 2500 | 269 | 0.108 |
| **2** | hydro-alcoholic | 2 | 0.2 | 2634 | 283 | 0.087 |
| **3** | hydro-alcoholic | 2 | 0.4 | 3664 | 264 | 0.077 |
| **4** | hydro-alcoholic | 2 | 0.6 | 3949 | 304 | 0.083 |
| | | | | | | |
| **5** | aqueous | 2 | 0 | 3200 | 225 | 0.070 |
| **6** | aqueous | 2 | 0.02 | 3305 | 215 | 0.065 |
| **7** | aqueous | 2 | 0.05 | 3100 | 218 | 0.071 |
| **8** | aqueous | 2 | 0.4 | 3556 | 260 | 0.073 |
| **9** | aqueous | 2 | 0.6 | 3573 | 243 | 0.068 |

The "hydro-alcoholic" preparation route referred to in **Table 4** refers to a water/1,2-propanediol mixture .

As can be taken from **Table 4,** for CHI/CNF hydrogel composites obtained from a hydro-alcoholic suspension **(Table 4,** entries 1-4), an increase in the G' value was observed with increased CNF content. While CHI/CNF hydrogels obtained from aqueous suspensions do not show a linear increase of the G' value with increased CNF content, the observed G' values for the composites measured are still high **(Table 4,** entries 5-9).

A reinforcement effect is thus observed with increasing cellulose nanofiber content, in both processing routes. In the case of hydro-alcoholic gels, the values of G"/G' slightly decrease with cellulose nanofiber content, displaying, with more accuracy than the absolute values of G', a more elastic behavior and reinforcement of the gel.

### Example 4: Suture test

The suturability of the hydrogel composites obtained from gelation of aqueous or hydro-alcoholic suspensions comprising different contents of dispersed cellulose fibers (CNFs) and chitosan (CHI) prepared as outlined in Example 1 was evaluated by a resistance test in the tear of the suture thread following the method described by Flamingo et al. (2016) (Flamingo et al. Biomacromolecules 2016, 17, 1662-1672). The method consists of making a loose suture in the hydrogel with a suture thread (Ethicon PDS II 4-0 (1,5 Ph.Eur.)) and gradually increasing the applied force to the suture till hydrogel break is achieved. The impact on the ultimate suture load of hydrogel composites obtained by the two different processing methods using either water/1,2-propanediol (hydro-alcoholic route) or only water (aqueous route) and using different cellulose nanofiber (CNF) contents was investigated.

The following **Table 5** shows the ultimate suture force and its value normalized by the hydrogel patch thickness observed for CHI/CNF hydrogel composites obtained from gelation of hydro-alcoholic and aqueous suspensions with different cellulose nanofiber contents.

**Table 5**

| **Entry** | **Nature of the suspension** | **CHI Content [wt.-%]** | **CNF Content [wt.-%]** | **Ultimate suture force [N]** | **Hydrogel patch thickness [mm]** | **Normalized ultimate suture force F/T [N.mm⁻¹]** |
|---|---|---|---|---|---|---|
| **1** | hydro-alcoholic | 2 | 0 | 0.032 | 2.00 | 0.016 |
| **2** | hydro-alcoholic | 2 | 0.2 | 0.112 | 2.00 | 0.056 |
| **3** | hydro-alcoholic | 2 | 0.4 | 0.070 | 2.10 | 0.033 |
| **4** | hydro-alcoholic | 2 | 0.6 | 0.153 | 2.00 | 0.077 |
| **5** | aqueous | 2 | 0 | 0.085 | 1.12 | 0.076 |
| **6** | aqueous | 2 | 0.2 | 0.105 | 1.21 | 0.087 |
| **7** | aqueous | 2 | 0.4 | 0.113 | 1.10 | 0.10 |
| **8** | aqueous | 2 | 0.6 | 0.144 | 1.00 | 0.14 |

As can be taken from **Table 5,** the normalized ultimate suture force was highest for the highest CNF content (CNF 0.6 wt.-%) (entry 4, 0.077 N.mm⁻¹; and entry 8, 0.14 N.mm⁻¹). The evolution of ultimate suture force increased with increased CNF content and this was more significant for the composites obtained by the aqueous route.

The suture tests thus demonstrate the ability of the hydrogel composites of the present invention for suturing, which is an essential parameter to evaluate the potential success for their application as implants for example for meniscus and cartilaginous tissue engineering.

### Example 5: Reestablishment of biomechanics in defect tissue

A hydrogel derived from a suspension obtained by the hydro-alcoholic route comprising chitosan at 3 wt.-% and cellulose nanofibers at 0.4 wt-% was implanted within a disc having an annulus fibrosus defect in order to reestablish the disc biomechanics.

Reestablishment of biomechanics was tested by a compression test. **Figure 2** shows the compression stress-strain curves observed as mechanical response of a healthy, a damaged and a hydrogel-implanted disc.

The linear elastic zone (EZ) of the curve of the hydrogel-implanted disc revealed a slope value similar to that of a healthy disc, and higher than that of a damaged disc. Besides, the stress values at failure of the healthy, hydrogel-implanted and untreated damaged discs were 6.7, 5.9 and 5.0 MPa, respectively. This demonstrates the fundamental role of hydrogel composites obtained from suspensions of the present invention for improving tissue biomechanics of lesioned discs.

## Claims

1. A suspension suitable for forming a hydrogel composite, the suspension comprising water (aqueous suspension) or a mixture of water and an alcohol (hydro-alcoholic suspension) as the solvent, cellulose nanofibers dispersed in said solvent, chitosan and an acid.

2. Suspension according to claim 1, wherein the content of the dispersed cellulose nanofibers is up to 5 wt.-%, preferably from 0.01 to 2 wt.-%, more preferably 0.02 to 0.6 wt.-%, and the concentration of chitosan is up to 15 wt.-%, preferably from 1 to 5 wt.-%, more preferably 2 to 4 wt.-%, each based on the total weight of the suspension

3. Suspension according to claim 1 or 2, wherein the chitosan has a molecular weight (Mw) from 400 to 800 kg/mol.

4. Suspension according to any of claims 1 to 3, wherein the pH is below 6.5, preferably from 3 to 5.

5. Suspension according to any of claims 1 to 4, wherein the suspension is a hydro-alcoholic suspension comprising a water/propanediol mixture as the solvent.

6. Suspension according to any of claims 1 to 5 for use in a method for tissue regeneration.

7. Suspension for use according to claim 6, wherein the tissue is native cartilaginous, meniscus or other connective tissue selected from tendon, dermis, and annulus fibrosus tissue.

8. Suspension for use according to according to claim 6 or 7, wherein the suspension is injected into an osteochondral defect or a meniscus/cartilage lesion or other connective tissue lesion to promote tissue regeneration.

9. Hydrogel composite obtainable by gelation of a suspension according to any of claims 1 to 5.

10. Hydrogel composite according to claim 9, wherein gelation is achieved by neutralizing the suspension.

11. Hydrogel composite according to claim 9 or 10, wherein gelation is achieved by adding NaOH or NH₃ to the suspension.

12. Hydrogel composite according to any of claims 9 to 11, wherein the hydrogel composite has an equilibrium storage modulus G' of at least 2600 Pa.

13. Hydrogel composite according to any of claims 9 to 12, wherein the hydrogel composite has a normalized ultimate suture force (ultimate suture force normalized by the hydrogel patch thickness) of at least 0.03 N.mm⁻¹.

14. Use of a hydrogel composite according to any of claims 9 to 13 for the manufacture of a bioresorbable and biocompatible implant.

15. Use according to claim 14, wherein the bioresorbable and biocompatible implant is a knitted textile implant.
